# EUROPEAN PATENT APPLICATION

(11) **EP 4 755 899 A1**
(43) Date of publication of application: **10.06.2026**
(21) Application number: 24849300.9
(22) Date of filing: 02.08.2024
(51) Int. Cl.: C07K 14/195, C12M 1/34, C12N 15/10, C12N 15/31, C12Q 1/04, G01N 33/571

(54) **IMMUNOASSAY METHOD USING SYPHILIS TREPONEMA PALLIDUM ANTIGEN 47 KDA (TPN47) AS ANTIGEN**

(30) Priority: 02.08.2023 JP 2023126558
(71) Applicant: Denka Company Limited, Tokyo 103-8338 (JP)
(72) Inventor: YANAGITA Tomoyo, Tokyo 103-8338 (JP); JARCZOWSKI Franziska, 06120 Halle/ Saale (DE); DIESSNER André, 06120 Halle/ Saale (DE)
(74) Representative: Hoffmann Eitle
(86) International application number: PCT/JP2024/027733
(87) International publication number: WO 2025/028655

(57) **Abstract**

This invention provides a method of immunoassay comprising detecting an antibody specific to *Treponema pallidum* in the serum with the use of the *Treponema pallidum* 47 kDa antigen (TpN47) as the antigen. This invention also provides a protein comprising a sequence deleted a part of the amino acid sequence of domain C of TpN47, and a method of immunoassay involving the use of such protein as the antigen.

## Description

### Technical Field

The present invention relates to an immunoanalytical method using the *Treponema pallidum* 47 kDa antigen (TpN47) as the antigen, a reagent used therefor, and a method using the reagent.

### Background Art

*Treponema pallidum* belongs to the bacterial family of spirochetes and is the causative pathogen of syphilis. Syphilis is primarily transmitted through sexual contact and may also be transmitted from the mother to the baby during pregnancy. The disease is characterized by distinct clinical stages and a long period of latent asymptomatic infection. Many people do not notice the symptoms and therefore remain unaware of their syphilis infection for years.

Effective treatments have been available since penicillin was introduced in the mid-20th century; however, syphilis remains a critical health problem around the world. In order to aid antibiotic therapy and prevent prevalence of syphilis, it is essential to identify patients infected with *Treponema pallidum.* In order to detect antibodies to *Treponema pallidum* in the serum, immunoassay or other diagnostic tools have been used.

Concerning serum antibodies after infection, the antibody titers specific to cardiolipin (tests using non-treponemal antigens: VDRL, the RPR card method, and the RPR automated method) are first elevated, and the antibody titers specific to *Treponema pallidum* (tests using *Treponema pallidum* antigens: the FTA-ABS method, the TPHA method, the TPPA method, and the TP antibody automated method) are then elevated. The anti-cardiolipin antibody titer is lowered in response to the treatment, and it is thus used for evaluation of treatment effects. However, cardiolipin is not a specific antigen, and, accordingly, biological false-positive reactions may occur. While the specificity of the anti-*Treponema pallidum* antibody assay is high, the antibody titer is gradually lowered but is continuously evaluated positive after the treatment. Accordingly, it is difficult to distinguish the latest syphilis infection from past syphilis infections.

### Citation List

### Patent Literature

Patent Literature 1: JP Patent Publication (Kokai) No. H09-288110 A

### Non Patent Literature

Non Patent Literature 1: V. Sambri et al., Clin. Diagn. Lab. Immunol., May 2001; 8 (3): 534-9

### Summary of Invention

### Technical Problem

An object of the present invention is to provide an immunoassay method for detecting a specific antibody to *Treponema pallidum* in the serum with the use of the *Treponema pallidum* 47 kDa antigen (TpN47) as the antigen. TpN47 is a giant protein consisting of 434 amino acid residues and is capable of detecting the anti-*Treponema pallidum* antibodies, which are IgG and IgM antibodies. In order to easily distinguish the latest syphilis infection from past syphilis infections, it is considered necessary to further enhance the assay sensitivity to IgM that is elevated at an early stage of syphilis infection. If the assay sensitivity to IgM that is elevated at an early stage of syphilis infection can further be enhanced, syphilis infection may be detected at an early stage. Accordingly, an object of the present invention is to provide an immunoassay method for detecting a IgM specific antibody to *Treponema pallidum* in the serum with the use of the *Treponema pallidum* 47 kDa antigen (TpN47) as the antigen.

### Solution to Problem

The present inventors have conducted concentrated studies. As a result, they discovered that deprivation of a part of domain C of the TpN47 antigen would enhance the reactivity with the anti-*Treponema pallidum* IgM antibody. This has led to the completion of the present invention.

Specifically, the present invention is as described below.
[1] A protein comprising an amino acid sequence of domain C of TpN47, comprising deletion of a part of the amino acid sequence.
[2] The protein according to [1], wherein the deletion of a part of the amino acid sequence of domain C of TpN47 is deletion of 110 to 127 amino acids on the C-terminal side.
[3] The protein according to [1], wherein the deletion of amino acids is deletion of amino acids 225 to 351, amino acids 226 to 351, amino acids 227 to 351, amino acids 228 to 351, amino acids 229 to 351, amino acids 230 to 351, amino acids 231 to 351, amino acids 232 to 351, amino acids 233 to 351, amino acids 234 to 351, amino acids 235 to 351, amino acids 236 to 351, amino acids 237 to 351, amino acids 238 to 351, amino acids 239 to 351, amino acids 240 to 351, amino acids 241 to 351, or amino acids 242 to 351 from the complete amino acid sequence of TpN47 shown in SEQ ID NO: 1.
[4] The protein according to [2] or [3] comprising the amino acid sequence shown in SEQ ID NO: 4.
[5] A TpN47 antigen comprising the protein according to any of [1] to [4].
[6] A method for measuring an anti-*Treponema pallidum* antibody by an immunoassay method with the use of the protein according to any of [1] to [4] or the TpN47 antigen according to [5] as an antigen.
[7] The method according to [6], comprising measuring the anti-*Treponema pallidum* IgM antibody with high sensitivity.
[8] The method according to [6] or [7], which is an agglutination method.
[9] A kit for assaying an anti*-Treponema pallidum* antibody by an immunoassay method, the kit comprising the protein according to any of [1] to [4] or the TpN47 antigen according to [5] as the antigen for measurement.
[10] The kit according to [9], wherein the immunoassay method is an agglutination method.

The description incorporates the contents disclosed by JP Patent Application No. 2023-126558, based on which the priority of the present application claims.

### Advantageous Effects of Invention

The anti-*Treponema pallidum* antibody may be measured with the use of the TpN47 antigen of the present invention, so as to measure the anti-*Treponema pallidum* IgM antibody with high sensitivity. This enables detection of syphilis infection at an early stage of infection with high sensitivity.

### Description of Embodiments

Hereafter, the present invention is described in detail. The unit "%" used herein is by mass (w/v%), unless otherwise specified.

The present invention relates to a protein in which a part of domain C of the *Treponema pallidum* 47 kDa surface antigen (TpN47) is deleted.

*Treponema pallidum* is known to comprise surface antigens with a wide variety of molecular weights on the cell surface thereof, and antigens with molecular weights of 47 kDa, 42 kDa, 17 kDa, and 15 kDa are known as major antigens. Such antigens are clearly separated from each other by electrophoresis. In addition to the gene encoding the 47 kDa surface antigen, the genes encoding 17 kDa and 15 kDa surface antigens have already been cloned, and antigens have been produced by genetic engineering. In addition, the amino acid sequences thereof have been determined.

SEQ ID NO: 1 shows the full-length amino acid sequence of TpN47, SEQ ID NO: 2 shows an amino acid sequence of TpN47 deleted the amino acid sequence (amino acids 1 to 19) of the signal peptide and the lipid-modified cysteine residue (amino acid 20) of the full length amino acid sequence of TpN47, SEQ ID NO: 3 shows the amino acid sequence of TpN47 deleted domain D (referred to as "domains ABC"), SEQ ID NO: 4 shows the amino acid sequence of TpN47 deleted a part of the amino acid sequence of domain C (referred to as "domains AB+D"), and SEQ ID NO: 5 shows the amino acid sequence of TpN47 deleted domains ABC (referred to as "domain D").

The crystalline structure or the biological role of TpN47 has been reported, and TpN47 is known to have 4 domains; i.e., domain A, domain B, domain C, and domain D. TpN47 consists of the amino acid sequence comprising: domain A consisting of amino acids 26 to 62 (amino acids 6 to 42 in the amino acid sequence shown in SEQ ID NO: 2) (domain A1) and amino acids 175 to 223 (amino acids 155 to 203 in the amino acid sequence shown in SEQ ID NO: 2) (domain A2); domain B consisting of amino acids 63 to 174 (amino acids 43 to 154 in the amino acid sequence shown in SEQ ID NO: 2); domain C consisting of amino acids 224 to 351 (amino acids 204 to 331 in the amino acid sequence shown in SEQ ID NO: 2), and domain D consisting of amino acids 352 to 434 (amino acids 332 to 414 in the amino acid sequence shown in SEQ ID NO: 2), from the N terminus of the complete amino acid sequence of TpN47 (SEQ ID NO: 1, Swiss-Prot P29723).

The TpN47 protein used as an antigen for measurement in the present invention does not comprise the amino acid sequence (amino acids 1 to 19) of the signal peptide and the lipid-modified cysteine residue (amino acid 20) in the full-length amino acid sequence of TpN47 shown in SEQ ID NO: 1.

The protein of the present invention has achieved enhanced reactivity with the anti-*Treponema pallidum* IgM antibody as a result of deletion of a part of the amino acid sequence of domain C of the TpN47 antigen. Even if a part of the amino acid sequence of domain C of TpN47 is deleted, measurement values are equivalent to the values attained with the use of TpN47 from which the amino acid sequence (amino acids 1 to 19) of the signal peptide and the lipid-modified cysteine residue (amino acid 20) have been removed. That is, it is possible to assay the anti-*Treponema pallidum* antibody with high sensitivity. Specifically, use of a protein deleted a part of the amino acid sequence of domain C of the *Treponema pallidum* 47 kDa surface antigen (TpN47) enables assays of the anti-*Treponema pallidum* IgM antibody with high sensitivity. Deletion of a part of the amino acid sequence of domain C of the TpN47 antigen is deletion of 110 to 127 amino acids, preferably 115 to 125 amino acids, more preferably 118 to 122 amino acids, and particularly preferably 120 amino acids from the C terminus of the amino acid sequence of domain C of the TpN47 antigen. The deletion of a part of the amino acid sequence is deletion of amino acids 225 to 351, amino acids 226 to 351, amino acids 227 to 351, amino acids 228 to 351, amino acids 229 to 351, amino acids 230 to 351, amino acids 231 to 351, amino acids 232 to 351, amino acids 233 to 351, amino acids 234 to 351, amino acids 235 to 351, amino acids 236 to 351, amino acids 237 to 351, amino acids 238 to 351, amino acids 239 to 351, amino acids 240 to 351, amino acids 241 to 351, or amino acids 242 to 351 (amino acids 205 to 331, amino acids 206 to 331, amino acids 207 to 331, amino acids 208 to 331, amino acids 209 to 331, amino acids 210 to 331, amino acids 211 to 331, amino acids 212 to 331, amino acids 213 to 331, amino acids 214 to 331, amino acids 215 to 331, amino acids 216 to 331, amino acids 217 to 331, amino acids 218 to 331, amino acids 219 to 331, amino acids 220 to 331, amino acids 221 to 331, or amino acids 222 to 331 in the amino acid sequence shown in SEQ ID NO: 2) from the complete amino acid sequence of TpN47 shown in SEQ ID NO: 1.

The amino acid sequences of SEQ ID NOs: 1 to 5 are shown below.
SEQ ID NO: 1 
MKVKYALLSAGALQLLVVGCGSSHHETHYGYATLSYADYWAGELGQSRDVLLAGN
AEADRAGDLDAGMFDAVSRATHGHGAFRQQFQYAVEVLGEKVLSKQETEDSRGRK
KWEYETDPSVTKMVRASASFQDLGEDGEIKFEAVEGAVALADRASSFMVDSEEYKIT
NVKVHGMKFVPVAVPHELKGIAKEKFHFVEDSRVTENTNGLKTMLTEDSFSARKVS
SMESPHDLVVDTVGTGYHSRFGSDAEASVMLKRADGSELSHREFIDYVMNFNTVRY
DYYGDDASYTNLMASYGTKHSADSWWKTGRVPRISCGINYGFDRFKGSGPGYYRLT
LIANGYRDVVADVRFLPKYEGNIDIGLKGKVLTIGGADAETLMDAAVDVFADGQPK
LVSDQAVSLGQNVLSADFTPGTEYTVEVRFKEFGSVRAKVVAQ
SEQ ID NO: 2 
GSSHHETHYGYATLSYADYWAGELGQSRDVLLAGNAEADRAGDLDAGMFDAVSR
ATHGHGAFRQQFQYAVEVLGEKVLSKQETEDSRGRKKWEYETDPSVTKMVRASASF
QDLGEDGEIKFEAVEGAVALADRASSFMVDSEEYKITNVKVHGMKFVPVAVPHELK
 
GIAKEKFHFVEDSRVTENTNGLKTMLTEDSFSARKVSSMESPHDLVVDTVGTGYHSR
FGSDAEASVMLKRADGSELSHREFIDYVMNFNTVRYDYYGDDASYTNLMASYGTK
HSADSWWKTGRVPRISCGINYGFDRFKGSGPGYYRLTLIANGYRDVVADVRFLPKYE
GNIDIGLKGKVLTIGGADAETLMDAAVDVFADGQPKLVSDQAVSLGQNVLSADFTP
GTEYTVEVRFKEFGSVRAKVVAQ
SEQ ID NO: 3 
GSSHHETHYGYATLSYADYWAGELGQSRDVLLAGNAEADRAGDLDAGMFDAVSR
ATHGHGAFRQQFQYAVEVLGEKVLSKQETEDSRGRKKWEYETDPSVTKMVRASASF
QDLGEDGEIKFEAVEGAVALADRASSFMVDSEEYKITNVKVHGMKFVPVAVPHELK
GIAKEKFHFVEDSRVTENTNGLKTMLTEDSFSARKVSSMESPHDLVVDTVGTGYHSR
FGSDAEASVMLKRADGSELSHREFIDYVMNFNTVRYDYYGDDASYTNLMASYGTK
HSADSWWKTGRVPRISCGINYGFDRFKGSGPGYYRLTLIANGYRDVVADVRFL
SEQ ID NO: 4 
GSSHHETHYGYATLSYADYWAGELGQSRDVLLAGNAEADRAGDLDAGMFDAVSR
ATHGHGAFRQQFQYAVEVLGEKVLSKQETEDSRGRKKWEYETDPSVTKMVRASASF
QDLGEDGEIKFEAVEGAVALADRASSFMVDSEEYKITNVKVHGMKFVPVAVPHELK
GIAKEKFHFVEDSRVTENTNGLKTMLTEDSFSARKVSSMESPHDLPKYEGNIDIGLKG
KVLTIGGADAETLMDAAVDVFADGQPKLVSDQAVSLGQNVLSADFTPGTEYTVEVR
FKEFGSVRAKVVAQ
SEQ ID NO: 5

PKYEGNIDIGLKGKVLTIGGADAETLMDAAVDVFADGQPKLVSDQAVSLGQNVLSA
DFTPGTEYTVEVRFKEFGSVRAKVVAQ

The protein of the present invention can be obtained by incorporating DNA encoding a protein comprising a sequence deleted a part of the amino acid sequence of domain C of TpN47 into a plasmid vector, introducing the plasmid vector into a host cell, and expressing the protein.

A host cell is not particularly limited, provided that it can produce the protein of the present invention. Examples of host cells include insect cells (e.g., Sf9 and Hi5 cells), *E. coli* cells, yeast cells (e.g., *Saccharomyces cerevisiae*, *Saccharomyces pombe*, *Pichia pastoris*), mammalian cells (e.g., CHO and HEK cells), and any other cells.

The protein of the present invention can be prepared with the use of a plant expression system or a genetically engineered plant. Protein production using the transient expression system in plants can be performed in accordance with a conventional technique. Examples of such techniques include, but are not particularly limited to, the agroinfiltration method, the plant virus vector method, the magnICON^{®} system, and the particle gun method.

When the agroinfiltration method is employed, *Agrobacterium* may be transformed with the vector, and a plant may then be infected with the *Agrobacterium* to obtain a plant that expresses the protein.

In the present invention, a protein in which a part of domain C of the *Treponema pallidum* 47 kDa surface antigen (TpN47) is deleted is referred to as the TpN47 antigen. Specifically, the present invention encompasses the TpN47 antigen consisting of a protein in which a part of domain C of the *Treponema pallidum* 47 kDa surface antigen (TpN47) is deleted.

In addition, the present invention encompasses a method for measurement and immunoanalysis of anti-TpN47 antibody with the use of a protein in which a part of domain C of the *Treponema pallidum* 47 kDa surface antigen (TpN47) is deleted as the antigen. Anti-TpN47 antibody measurement enables detection of the presence or absence of *Treponema pallidum* in a test sample obtained from a subject. By detecting the presence or absence of *Treponema pallidum* in a test sample, whether or not the subject is infected with *Treponema pallidum*; i.e., whether or not the subject has syphilis, can be determined, or auxiliary data used for diagnosis thereof can be obtained. With the use of a protein in which a part of domain C of the *Treponema pallidum* 47 kDa surface antigen (TpN47) is deleted as the antigen, the anti-*Treponema pallidum* TpN47 IgM antibody that appears at an early stage of infection can be detected with high sensitivity, and syphilis can be detected at an early stage of infection.

The present invention can be applied to any immunological measurement methods that utilize the antigen-antibody reactions. Specifically, the present invention can be applied to, for example, immunodiffusion, immunoturbidimetry, immunonephelometry, hemagglutination, the latex method, enzyme immunoassay (EIA), radioimmunoassay (RIA), or immunochromatography. In particular, the present invention exerts remarkable effects in the agglutination methods, such as immunoturbidimetry, immunonephelometry, hemagglutination, and the latex agglutination method.

The agglutination method is an immunoassay method involving the use of insoluble carriers, such as latex particles, bentonite, collodion, kaolin, fixed sheep red blood cells, and the like. Insoluble carriers are preferably latex particles, and examples of latex particles that can be used include polystyrene latex particles, styrene-butadiene copolymer latex particles, and polyvinyl toluene latex particles. In immunoassays using the agglutination method, insoluble carriers may be sensitized with antigens; that is, the antigens may be bound to the surface of the carriers.

The immunoassay method of the present invention is preferably performed with the use of an automated analyzer. An automated analyzer is used for biochemical testing that can be applied to an immunoassay method, which comprises a mechanism of dispensing a reagent into a reaction vessel through a tube by a continuous dispensing system. More specifically, such automated analyzer comprises a means for providing a reagent inside or outside the apparatus and it continuously dispenses a reagent through a tube into a reaction vessel provided in the apparatus. The reagent is dispensed through the tube with the aid of a pump, such as a peristaltic pump, in combination with a switch valve. Examples of such apparatuses includes various commercially available apparatuses, such as Hitachi Automated Analyzer 7180, Hitachi Automated Analyzer 7250, Hitachi Automated Analyzer 7450, and Hitachi Automated Analyzer 7350 (Hitachi High-Tech Corporation).

A method of measurement performed with the use of an automated analyzer comprises optically capturing agglutinates resulting from bond formation between the antigen-sensitized carriers and the target antibodies in a reaction vessel and measuring the antibodies. Specifically, agglutinates are irradiated with lights in visible to near-infrared ranges, and a change in the absorbance or a change in the scattered light intensity is detected to measure the extent of agglutination of carrier particles. Examples of methods of measurement include the one-point method, the two-point method, the three-point method, and the rate method, and the method of the present invention can be applied to any of such methods of measurement. A test sample is dispensed into a reaction vessel in advance, the first reagent and the second reagent are dispensed thereinto, the reaction is allowed to proceed, and the extent of agglutination is measured. Thus, the target antigen or the target antibody in the test sample can be quantified using the extent of agglutination as the indicator. The order to add the first reagent and the second reagent is not limited. It is preferable that the first reagent be dispensed before the second reagent or the first reagent and the second reagent be dispensed simultaneously. In such a case, a standard curve relating the concentration of the target analyte to the extent of agglutination may be created in advance using a plurality of samples with known concentration of the target analyte, and the concentration of the target analyte in the test samples can be calculated based on the standard curve.

The first reagent may contain a buffer. The first reagent may contain another stabilizer reagent, such as a surfactant, a protective reagent, and the like. The second reagent may contain the particle sensitized with the antigen that binds specifically to the analyte antibody, and the second reagent may contain another stabilizer reagent, such as a surfactant, a protective reagent, and the like.

A method for sensitizing a carrier with an antigen may be performed in accordance with a conventional technique without particular limitation. For example, an antigen may be physically adsorbed to or chemically bound to a carrier. More specifically, for example, an antigen may be mixed with a carrier, and the mixture may be heated with shaking at 30°C to 37°C for 1 to 2 hours to sensitize the carrier with the antigen. The amount of the antigen to sensitize the carrier can be appropriately determined in accordance with the particle diameter of the carrier used. After the carrier is sensitized with the antigen, an unsensitized region on the carrier surface is preferably blocked with, for example, bovine serum albumin, human serum albumin, rabbit serum albumin, or egg albumin. The antigen-sensitized carrier is preferably retained in the form of a medium dispersion before the reaction with the test sample. Examples of media that can be used herein include phosphate buffer and glycine buffer. A medium may be supplemented with bovine serum albumin, gelatin, gum Arabic, or the like, according to need. The antigen-sensitized carrier thus prepared is allowed to react with the test sample, the reactivity between the sensitizing antigen and the antibody in the test sample is determined based on the presence or absence or the extent of agglutination, and the antibody in the test sample can be detected.

In the agglutination method using sensitized particles, detection is performed by adding several tens to several hundreds of µl of physiological saline or an adequate buffer to several to several tens of µl of the test sample, mixing the mixture in an adequate reaction vessel, performing incubation for several minutes, and adding several tens to several hundreds of µl of antigen-bound sensitized particles, followed by incubation for several minutes. Subsequently, the absorbance may be measured at an adequate wavelength (e.g., 570 nm) to measure the turbidity caused by agglutination of sensitized particles resulting from the antigen-antibody reaction. Alternatively, several tens to several hundreds of µl of a solution of sensitized particles suspended in a buffer may be added to several µl of a reaction sample. Examples of buffers include adequate buffers with pH 5.0 to 10, such as phosphate buffer, borate buffer, and Tris buffer.

The test samples subjected to the immunoassay method of the present invention are not particularly limited, body fluid samples, such as blood, serum, plasma, urine, stool, saliva, interstitial fluid, spinal fluid, and swab specimen samples, or a diluted product thereof are preferable, and blood, serum, and plasma samples or a diluted product thereof are more preferable.

In the agglutination method, the first reagent may be mixed with the second reagent containing latex particles.

The present invention further encompasses an immunoassay kit comprising the assay reagent. The immunoassay kit comprises a protein in which a part of the amino acid sequence of domain C of TpN47 is deleted as an antigen for measurement. When the buffer composition does not contain antigen-sensitized particles, the immunoassay kit may further comprise antigen-sensitized particles separately, and, furthermore, the kit may comprise a positive control, a negative control, instructions, and the like.

### Examples

The present invention is described in greater detail with reference to the following examples, although the present invention is not limited to these examples.

### (1) Preparation of TpN47

TpN47 was prepared using the transient expression system in plants. TpN47 was cloned into the tobacco mosaic virus (TMV) vector (Icon Genetics). The vector was transformed into an *Agrobacterium* cell, the transformed cell was cultured, the mixed culture was infiltrated into *Nicotiana benthamiana* leaves, the leaves were harvested approximately 1 week later, and the harvested leaves (infected leaves) were then frozen.

The frozen infected leaves (200 g) were separated by weighing and ground using a cutter mixer. An extraction solution (200 ml, 100 mM Tris, 250 mM NaCl, 5 mM EDTA, 40 mM sodium ascorbate) was added to the ground leaves, and the infected leaves were repeatedly ground. The supernatant was collected by centrifugation. The collected solution was filtered through a 0.22-µm filter. The filtrate was subjected to affinity column chromatography to remove impurities.

In accordance with the method described above, TpN47 consisting of domains ABCD (SEQ ID NO: 2), TpN47 consisting of domains ABC without domain D (SEQ ID NO: 3), TpN47 consisting of domains AB+D without a part of domain C (SEQ ID NO: 4), and TpN47 consisting of domain D without domains ABC (SEQ ID NO: 5) were expressed and prepared.

TpN15 and TpN47 were prepared in the same manner. The amino acid sequences of TpN15 and TpN47 and the DNA sequences encoding the same are known.

### (2) Preparation of first reagent

A surfactant and bovine serum albumin were added to a buffer (Tris, pH 8.0) to prepare a first reagent.

### (3) Preparation of second reagent

With the use of domains ABCD, domains ABC, domains AB+D, and domain D of TpN47, TpN15, and TpN47 prepared in (1), assay reagents used for immunoagglutination were prepared in the manner described below.

Sensitized particles comprising domains ABCD, domains ABC, domains AB+D, and domain D of TpN47, TpN15, and TpN47 prepared in (1) supported thereon in an amount of 3.9 mg relative to 1 ml of a polystyrene latex suspension were suspended in a buffer (Bis-Tris, pH 7.0) to 0.12% to prepare a second reagent.

### (4) Measurement using automated analyzer

Measurement was performed automatically by an end-point assay method using Hitachi Automated Analyzer 7180 (Hitachi High-Tech Corporation).

With the use of the first reagent and the second reagent comprising the sensitized particles comprising domains ABCD, domains ABC, domains AB+D, and domain D of TpN47, serum specimens were assayed. The first reagent (100 µl) was added to 8.5 µl of the specimen solution, and the mixture was mixed at 37°C with stirring. The mixture was allowed to stand for 5 minutes, 50 µl of the second reagent was added thereto, and the mixture was further mixed at 37°C with stirring. The result of the agglutination reaction performed for approximately 5 minutes was assayed as the change in the absorbance, and the anti-*Treponema pallidum* antibody concentration of each specimen was determined based on the calibration curve prepared using the standard solution (HEPES buffer, bovine serum albumin, sodium chloride, the anti-*Treponema pallidum* antibody, pH 7.4).

### (5) Comparison of reactivity of TpN47

The measurement values of the reagents using domains ABCD, domains ABC, domains AB+D, and domain D of TpN47 prepared in (1) relative to the standard specimens were compared.

With the use of the serum specimens (2 specimens) obtained from syphilis patients as the specimens, the dilution series of the standard solution (HEPES buffer, bovine serum albumin, sodium chloride, the anti-*Treponema pallidum* antibody, pH 7.4) was prepared. Each of the diluted specimens was measured by the method described in (4) with the use of the first reagent prepared in (2) and the second reagent using domains ABCD, domains ABC, domains AB+D, and domain D of TpN47 prepared in (3).

The results are shown in Table 1.

**[Table 1]**

| Standard solution (U/ml) | Measurement value (ΔAbs x 10,000) | | |
|---|---|---|---|
| | ABCD | AB+D | D |
| 0 | -38 | -31 | -21 |
| 20 | 64 | 53 | -16 |
| 50 | 301 | 164 | 8 |
| 150 | 1140 | 767 | 64 |
| 300 | 1992 | 1613 | 120 |
| 500 | 2373 | 2128 | 177 |

The results shown in Table 1 demonstrate that, in comparison with the reagent using TpN47 consisting of domains ABCD and the reagent using TpN47 consisting of domains AB+D without a part of domain C, the reagent using TpN47 consisting of domain D without domains ABC exhibits lower reactivity to the standard solution.

In addition, the rabbit immune serum to TpN47 was subjected to the measurement in accordance with the method described in (4) as the specimen with the use of the first reagent prepared in (2) and the second reagent using domains ABCD, domains ABC, domains AB+D, and domain D of TpN47 prepared in (3).

The results are shown in Table 2.

**[Table 2]**

| | Measurement value (ΔAbs x 10,000) | | |
|---|---|---|---|
| | ABCD | AB+D | D |
| Rabbit immune serum reacting with TpN47 | 2229 | 2123 | -8 |

The results shown in Table 2 demonstrate that, in comparison with the reagent using TpN47 consisting of domains ABCD and the reagent using TpN47 consisting of domains AB+D without a part of domain C, the reagent using TpN47 consisting of domain D without domains ABC exhibits lower reactivity to the rabbit immune serum to TpN47.

### (6) Comparison of measurement values to TpN47 serum specimens

The measured anti-TP antibody titers of the reagents using TpN47 prepared in (1) reacting with the positive serum specimens were compared.

The two serum specimens (Serum 1 and Serum 2) were assayed by the method described in (4) with the use of the first reagent prepared in (2) and the second reagent using domains ABCD, domains ABC, domains AB+D, and domain D of TpN47 prepared in (3).

The results are shown in Table 3.

**[Table 3]**

| Serum specimens | Anti-TP antibody titer (U/ml) | | |
|---|---|---|---|
| | ABCD | ABC | AB+D |
| Serum 1 | 6.5 | 2.5 | 12.3 |
| Serum 2 | 4.1 | 0.4 | 18.8 |

The results shown in Table 3 demonstrate that, in comparison with the reagent using TpN47 consisting of domains ABCD and the reagent using TpN47 consisting of domains ABC without domain D, the reagent using TpN47 consisting of domains AB+D without a part of domain C exhibits higher reactivity and higher sensitivity to the positive serum specimens.

In addition, anti-TpN15, anti-TpN47, and anti-TpN47 IgG and IgM antibodies contained in the serum specimens 1 and 2 were assayed with the use of *recom*Line Treponema IgG (tradename, commercially available from MIKROGEN) and *recom*Line Treponema IgM (tradename, commercially available from MIKROGEN) to detect the anti-*Treponema pallidum* antibody in human serum samples. Evaluation as to the presence or absence of antibodies to each of the *Treponema pallidum* antigens (TpN47, TpN47, and TpN15) and evaluation as to the intensities of the detected bands were made in accordance with the standards shown in Table 4 in comparison with cutoff bands on each of test papers.

**[Table 4]**

| Evaluation of band intensity in association with cutoff band | |
|---|---|
| Band staining intensity | Score |
| No reactivity | 0 |
| Very low intensity (lower than the cutoff band) | 0.5 |
| Low intensity (equal to the cutoff band) | 1 |
| High intensity (higher than the cutoff band) | 2 |
| Very high intensity | 3 |

The results are shown in Table 5.

**[Table 5]**

| | | Anti-TpN15 antibody | Anti-TpN17 antibody | Anti-TpN47 antibody |
|---|---|---|---|---|
| Serum 1 | IgG | 0 | 0 | 0 |
| | IgM | 0 | 1 | 1 |
| Serum 2 | IgG | 0 | 0 | 0 |
| | IgM | 0 | 0 | 1 |

The results shown in Table 5 demonstrate that the anti-TpN47 IgM antibody is detected in both the serum specimens 1 and 2.

The results shown above indicate that the protein of the present invention in which a part of domain C of TpN47 is deleted reacts with the IgM antibody with high sensitivity.

### (7) Comparison with products of other manufacturers

With the use of the first reagent prepared in (2) and the second reagent prepared in (3) using domains AB+D of TpN47 deleted of a part of domain C (the present invention), the measured anti-TP antibody titers of 3 products of other manufacturers (Other products 1 to 3) were compared.

The reagent of the present invention was measured by the method described in (4).

As the specimens, the syphilis seroconversion panel (tradename, commercially available from SeraCare), which is a set of plasma samples obtained from syphilis patients with the elapse of time, was used.

The plasma samples obtained with the elapse of time were assayed with the use of *recom*Line Treponema IgG (tradename, commercially available from MIKROGEN) and *recom*Line Treponema IgM (tradename, commercially available from MIKROGEN) to detect the anti-*Treponema pallidum* antibody in the human serum samples. Evaluation as to the presence or absence of antibodies to each of the *Treponema pallidum* antigens and evaluation as to the intensities of the detected bands were made in accordance with the standards shown in Table 4 in comparison with cutoff bands on each of test papers.

The results are shown in Table 6.

**[Table 6]**

| Days since 1st bleed | Panel member | Present invention | Other product 1 | Other product 2 | Other product 3 | IgM | | | IgG | | |
|---|---|---|---|---|---|---|---|---|---|---|---|
| | | | | | | TpN47 | TpN17 | TpN15 | TpN47 | TpN17 | TpN15 |
| 0 | PSS901-01 | Negative | Negative | Negative | Negative | 0 | 0 | 0 | 0 | 0 | 0 |
| 5 | PSS901-02 | Negative | Negative | Negative | Negative | 0 | 0 | 0 | 0 | 0 | 0 |
| 10 | PSS901-03 | Negative | Negative | Negative | Negative | 0 | 0 | 0 | 0 | 0 | 0 |
| 13 | PSS901-04 | Negative | Negative | Negative | Negative | 0 | 0 | 0 | 0 | 0 | 0 |
| 31 | PSS901-05 | Positive | Negative | Negative | Negative | 1 | 1 | 0 | 0 | 0 | 0 |
| 45 | PSS901-06 | Positive | Negative | Negative | Positive | 1 | 1 | 0 | 1 | 0.5 | 0.5 |
| 48 | PSS901-07 | Positive | Negative | Negative | Positive | 1 | 1 | 0 | 1 | 0.5 | 0.5 |
| 52 | PSS901-08 | Positive | Negative | Positive | Positive | 2 | 2 | 0.5 | 2 | 1 | 1 |
| 59 | PSS901-09 | Positive | Positive | Positive | Positive | 2 | 2 | 1 | 3 | 2 | 2 |

The results shown in Table 6 demonstrate that the reagent using TpN47 consisting of domains AB+D while being deprived of a part of domain C enables detection of the anti-*Treponema pallidum* antibody in the plasma specimens obtained from syphilis patients on Day 31. In contrast, the other products did not enable detection of the anti-*Treponema pallidum* antibody in the plasma specimens obtained from syphilis patients until Day 45. This indicates that the reagent using TpN47 consisting of domains AB+D while being deprived of a part of domain C enables detection of the anti-*Treponema pallidum* antibody earlier than the other products.

In addition, the results of detection of the anti-*Treponema pallidum* antibodies in the serum specimens demonstrate the presence of the IgM antibody to TpN47 on Day 31.

The results shown above indicate that the protein of the present invention in which a part of domain C of TpN47 is deleted reacts with the anti-TpN47 IgM antibody that appears at a very early stage with high sensitivity and assays the antibody in the plasma specimens obtained from syphilis patients at an earlier stage than the other products.

### Industrial Applicability

The method of the present invention enables detection of syphilis infection at an early stage of infection.

### Sequence Listing Free Text

### SEQ ID NOs: 2 to 5: Synthetic

All publications, patents, and patent applications cited herein are incorporated herein by reference in their entirety.

## Claims

1. A protein comprising an amino acid sequence of domain C of TpN47, comprising deletion of a part of the amino acid sequence thereof.

2. The protein according to claim 1, wherein the deletion of a part of the amino acid sequence of domain C of TpN47 is deletion of 110 to 127 amino acids on the C-terminal side.

3. The protein according to claim 1, wherein the deletion of amino acids is deletion of amino acids 225 to 351, amino acids 226 to 351, amino acids 227 to 351, amino acids 228 to 351, amino acids 229 to 351, amino acids 230 to 351, amino acids 231 to 351, amino acids 232 to 351, amino acids 233 to 351, amino acids 234 to 351, amino acids 235 to 351, amino acids 236 to 351, amino acids 237 to 351, amino acids 238 to 351, amino acids 239 to 351, amino acids 240 to 351, amino acids 241 to 351, or amino acids 242 to 351 from the complete amino acid sequence of TpN47 shown in SEQ ID NO: 1.

4. The protein according to claim 2 comprising the amino acid sequence shown in SEQ ID NO: 4.

5. The protein according to claim 3 comprising the amino acid sequence shown in SEQ ID NO: 4.

6. A TpN47 antigen comprising the protein according to any one of claims 1 to 5.

7. A method for measuring an anti-*Treponema pallidum* antibody by an immunoassay method with the use of the protein according to any one of claims 1 to 5 as an antigen.

8. A method for measuring an anti-*Treponema pallidum* antibody by an immunoassay method with the use of the TpN47 antigen according to claim 6 as an antigen.

9. The method according to claim 7, comprising measuring an anti-*Treponema pallidum* IgM antibody with high sensitivity.

10. The method according to claim 8, comprising measuring an anti-*Treponema pallidum* IgM antibody with high sensitivity.

11. The method according to claim 7, which is an agglutination method.

12. The method according to claim 8, which is an agglutination method.

13. The method according to claim 9, which is an agglutination method.

14. The method according to claim 10, which is an agglutination method.

15. A kit for measuring an anti-*Treponema pallidum* antibody by an immunoassay method, the kit comprising the protein according to any one of claims 1 to 5 as an assay antigen.

16. A kit for measuring an anti-*Treponema pallidum* antibody by an immunoassay method, the kit comprising the TpN47 antigen according to claim 6 as an assay antigen.

17. The kit according to claim 15, wherein the immunoassay method is an agglutination method.

18. The kit according to claim 16, wherein the immunoassay method is an agglutination method.
